# EUROPEAN PATENT SPECIFICATION

(11) **EP 2 775 905 B1**
(45) Date of publication and mention of the grant of the patent: **14.04.2021**
(21) Application number: 12837614.2
(22) Date of filing: 08.11.2012
(51) Int. Cl.: A61B 5/022

(54) **A WEAREABLE TONOMETER STRUCTURE**
TRAGBARE TONOMETERSTRUKTUR
STRUCTURE DE TONOMÈTRE PORTABLE

(30) Priority: 08.11.2011 IT PI20110127
(43) Date of publication of application: 17.09.2014
(73) Proprietor: AB Medica S.p.A., 20124 Milano (MI) (IT)
(72) Inventor: VALDASTRI, Pietro, I-56023 Navacchio (PI) (IT); NANNIZZI, Martina, I-55015 Montecarlo (LU) (IT); DE NEGRI, Ferdinando, I-Pisa 56124 (IT)
(74) Representative: Concone, Emanuele
(86) International application number: PCT/IB2012/056251
(87) International publication number: WO 2013/068955

(56) References cited:
- US-A- 5 243 992
- US-A- 5 494 043
- US-A1- 2011 077 537

## Description

### Field of the invention

The present invention relates to the medical field, and, in particular, it relates to a tonometer structure that can be worn by a user for a permanent and non invasive monitoring of a patient's artery path, e.g. the radial artery.

Furthermore, the invention relates to a corresponding measurement method that can be carried out by the above tonometer structure.

### State of the art

Arterial pressure is one of the most useful parameters to diagnose a cardiovascular disease and to follow-up patients who suffer from these pathologies. The measurement methods are different in nature and type, and it is possible to classify the methods into two main groups, i.e. invasive methods and non-invasive methods. The invasive methods allow permanently, beat-to-beat measuring the sphygmic signal, but they require introducing a needle into the artery, with drawbacks that such a technique of the type entails. The non-invasive methods may essentially be of two main types, i.e. direct measurement methods and derivative measurement methods. Direct measurements comprise using instruments that measure the arterial pressure in a non-permanent way, for instance by oscillometric techniques or by auscultation techniques, via a pneumatic cuff, which is very annoying to wear. The derivative measurements use non invasive instruments configured to obtain permanent parameter signals related to the arterial pressure from non-sphygmic signals. For instance, instruments are used based on the pulse wave analysis, which exploits the ECG and plethysmographic signals. Since they provide values deriving from non-sphygmic signals, these instruments may introduce errors during the signal transformation.

A tonometer is a non-invasive apparatus for measuring the arterial pressure by pressing an artery path, for example the radial artery path, pressing it against a bone structure laying below. Then, by a dedicated algorithm, the central arterial pressure and the central mean aortic pressure, systolic and diastolic, are derived from it.

Among the earliest prior art tonometers, non-wearable tonometers are known, as disclosed in US5363855, US5605156 and US6290650.

A wearable clock-shaped tonometer is disclosed in IL166200.

This device is comfortable to wear and allows monitoring a patient's pressure even for a long time and even far away from a specialized centre and without the support of a qualified person.

In particular, the device of IL166200 comprises a pressure sensor that is immersed in a chamber containing a transmission fluid, such as a gel, and the chamber can contact the artery and with the transmission fluid, and transmits forces that arise from the deformation of the artery. Opposite to the sensor chamber, a display unit is provided by which the data measured by the sensor can be displayed.

However, this device has a drawback. By using only one pressure sensor, a loss or a reduction of the measurement signal occurs, since the position of the sensor with respect to the artery may change while the device is being worn by the user.

In order to overcome this drawback, devices have been developed that provide more pressure sensors arranged adjacent with respect to each other, as disclosed in US2009069698. In this case, a dedicated control unit identifies the sensor that provides the best signal, according to the intensity and to the quality of the signals received from each sensor, and performs the measurement through the sensor so identified. This way, the positioning is optimised, since the sensor is selected that is best positioned upon the artery. In this case, optical sensors are used.

It has also been observed that each sensor is affected by systematic errors during the signal measurement, which is due to different factors, for example to the user's movement, to the position of the tonometer on the artery, etc.

However, in the case of a wearable tonometer device that may be displaced and/or may be undergo impacts, the array of sensors may be moved in such a way that the selected sensor may lose the signal and may become a sensor incorrectly positioned with respect to the artery.

In Edward J. Ciaccio and Gary M. Drzewiecki "Tonometric arterial pulse sensor with noise cancellation" IEEE Transactions on Biomedical Engineering, vol. 55, no. 10, October 2008, a device is described for permanently non-invasively monitoring the pressure wave of an artery. In particular, two piezoelectric sensors are provided in order to eliminate artifacts that reduce the quality of the signal, in particular movement artifacts and background noise. A first sensor is located at the radial artery (p) and another sensor is positioned in such a way that to prevent any overlapping with the artery pulsation (n) . A step of noise removal is conventionally carried out using a reference input, or reducing the movement and noise artifacts from the acquired artery pulsation tonometric signal.

Even in this case, the arrangement of the sensor upon the artery must be carried out with precision, so that the received measurement signal is strong enough.

In US2010/204588 an apparatus and a method are described for identifying an ideal blood pressure measurement site. In particular, the apparatus comprises a detection unit for detecting the pressures that are exerted to a blood vessel in a particular region of the body, a calculation unit to calculate the waveform that represents the detected signal, and a decision unit for deciding whether the examined site is the ideal site. The detection unit comprises an oscillometric sensor, i.e. a spot-type pressure sensor to be used together with a pressurization unit, such as a pneumatic cuff.

The step of checking check whether the examined site is the ideal blood pressure measurement site is carried out by analysing the trend of the detected pressures, which are plotted accordingly.

Thus, the solution proposed in US2010/204588 was conceived to identify the ideal site where to measure the arterial pressure, which will take place later by a different and dedicated instrument. Therefore, the solution proposed in US2010/204588 is not well-suited to measure the arterial pressure value, nor can it carry out a permanent monitoring of a patient's arterial pressure, during a determined time. Moreover, the processing of the measured data is complex and cannot provide precise pressure data.

In US6475153 a method is described for obtaining pressure data by means of optical sensors. Once the patient's pressure have been obtained, the linear calibration relationships between the output signal and the blood pressure are determined by a calibration procedure that is carried out for some of the optical sensors of by the device.

In particular, the solution described in US6475153 comprises using non optical auxiliary sensors for eliminating the artifacts, since the optical sensors cannot detect the so-called "hold-on pressure" artifacts.

Therefore, also the solution of US6475153 necessarily comprises a complex processing of the measured data, in particular, in order to eliminate the artifacts, and cannot provide precise pressure data.

### Summary of the invention

It is therefore a feature of the invention to provide a wearable tonometer structure for obtaining precise and reliable measurement signal, regardless of the movement that may be experienced by a measurement sensor optimally positioned with respect to an artery.

It is another feature of the invention to provide a wearable tonometer structure that makes it possible to obtain a more precise and more reliable measurement signal, without errors or background noise.

These and other objects are achieved by a tonometer structure according to claim 1.

Further advantages are achieved through the tonometer strucutures according to the dependent claims.

### Brief description of the drawings

The invention will now be shown with the following description of its exemplary embodiments, exemplifying but not limitative, with reference to the attached drawings in which:
- Fig. 1 is a perspective view of an arrangement of the tonometer structure, according to the invention, above a wrist radial artery, the tonometer structure comprising a plurality of pressure sensors;
- Fig. 1A is a diagram schematically showing the measurement signals associated with the sphygmic waves detected by the piezoresistive sensors, according to the invention;
- Figs. 1B to 1D are block diagrams schematically showing the logical measurement sequence followed by the tonometer structure of Fig. 1, according to three possible alternative exemplary embodiments of the present invention, in particular the block diagrams point out the recognition sequence, repeated at determined time intervals, of a reference signal that turns out to be correctly positioned above the radial artery, as well as the recognition of boundary signals that are computed in order to improve the quality of the reference signal and to eliminate any artifacts due to background noise;
- Figs. 2 and 3 show a first exemplary embodiment of the detection unit, in which the detection unit is arranged upon a flexible support body that can be arranged about a user's wrist (Fig.3);
- Fig. 4 shows a perspective view of a further exemplary embodiment of a tonometer structure comprising a plurality of housings for each sensor that forms the sensor array; this figure also shows the introduction of a sensor in its own housing;
- Fig. 5 shows a perspective view of a second exemplary embodiment of a tonometer structure comprising a single housing for sensors arrays that are arranged above a sensorized sheet comprising a single PCB for the whole array;
- Fig. 6 shows a perspective view of the exemplary embodiment of Fig. 5, during the introduction of the sensorized sheet into the housing;
- Fig. 7 shows a diagrammatical view of the electronic architecture that forms the tonometer structure;
- Fig. 7A shows a diagrammatical view of the electronic architecture that forms the tonometer structure of Fig. 7, also comprising a user interface provided with a display unit;
- Fig. 8 shows a diagrammatical view of the electronic architecture that forms the tonometer structure of Fig. 5 also comprising a wireless transmission means, along with a remote unit;
- Figs. 9 to 15 show an exemplary embodiment of the tonometer device of Figs. 2 to 6;
- Figs. 16 to 19 show a further possible exemplary embodiment of the tonometer device, according to the present invention, equipped in particular with a means for adjusting the distance from the artery path.

### Detailed description of the invention

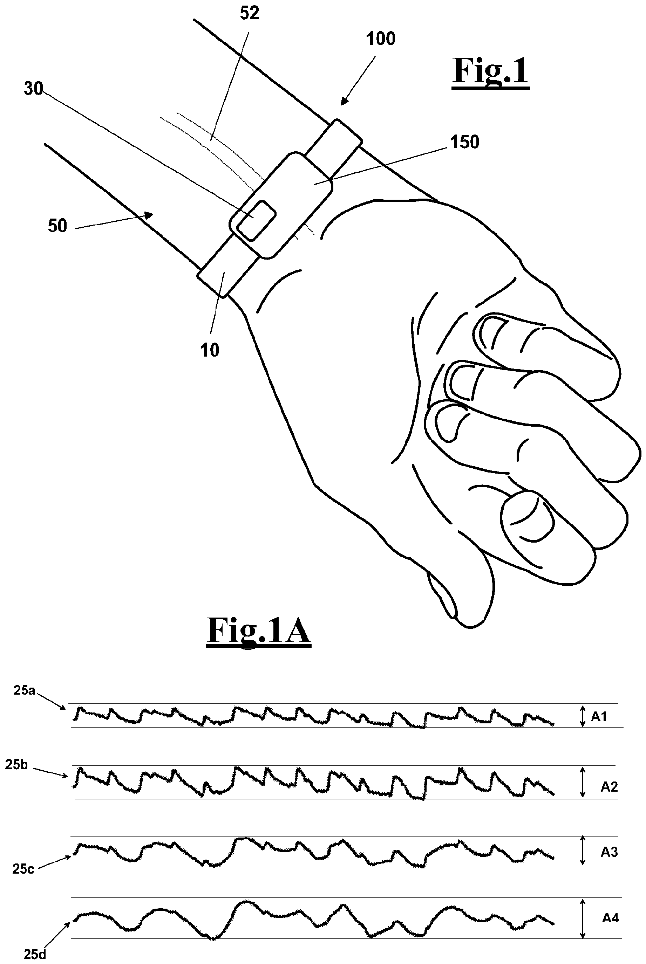

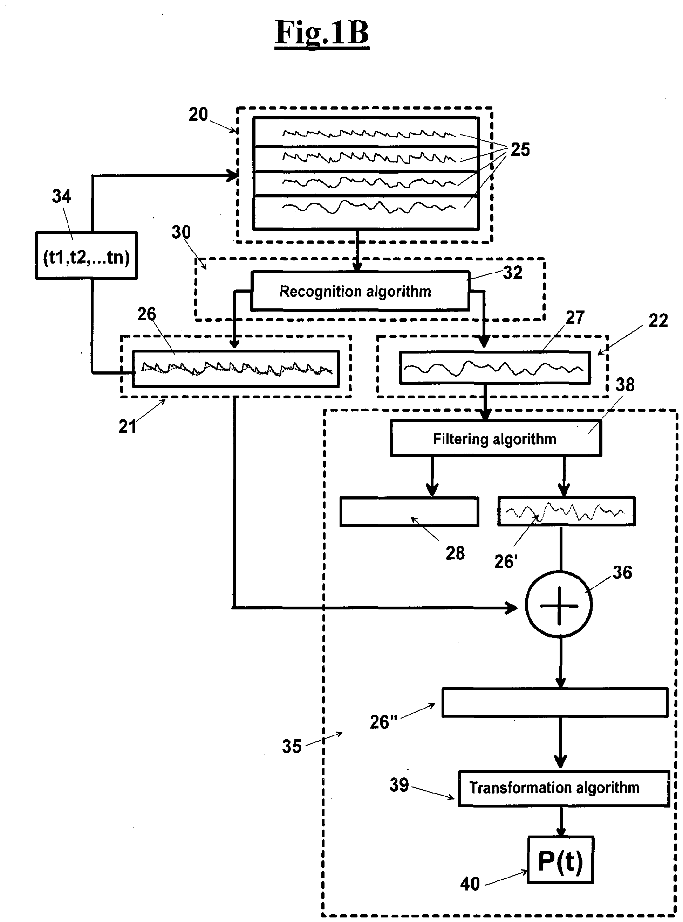

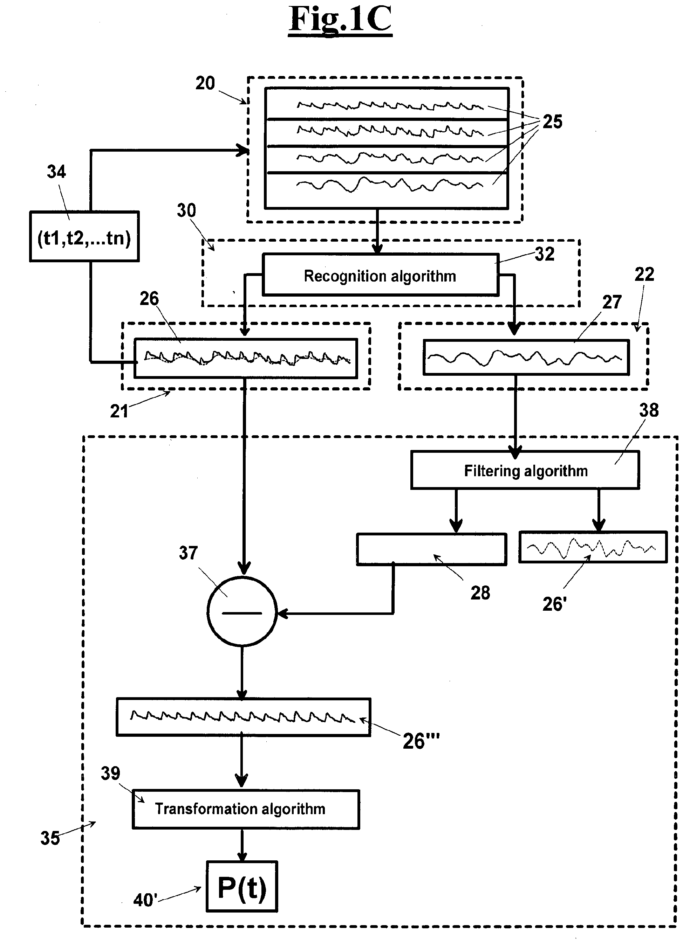

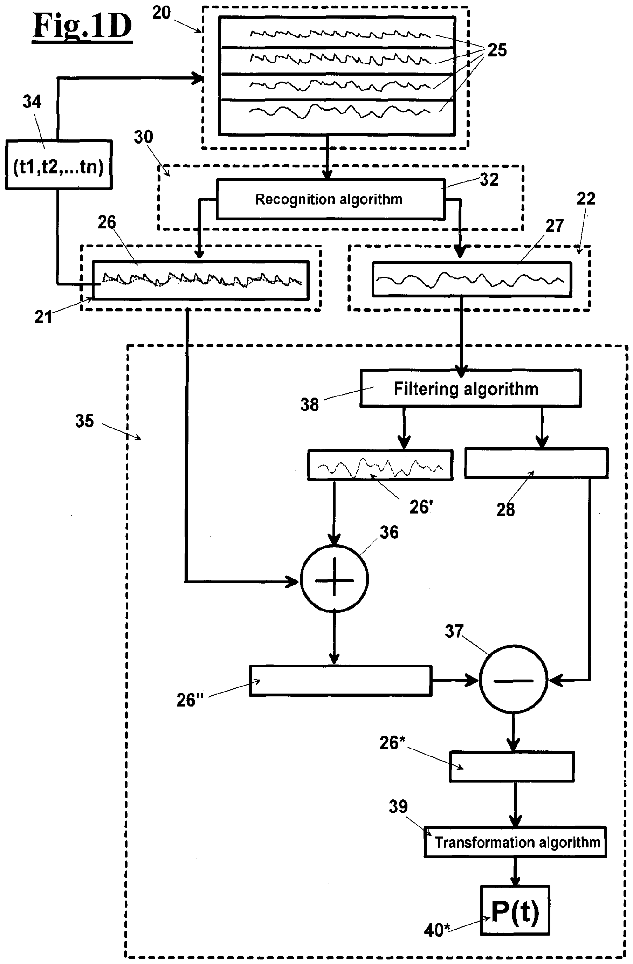

With reference to Fig. 1, a tonometer structure 100, according to the invention, comprises a support body 10 that, in use, is worn by a patient 50. Support body 10 comprises, in particular, a detection unit 150 configured to be arranged, in use, in contact with the skin of patient 50 proximate to an artery path 52, in order to measure the pressure variation which occur in artery path 52, due to the blood flow.

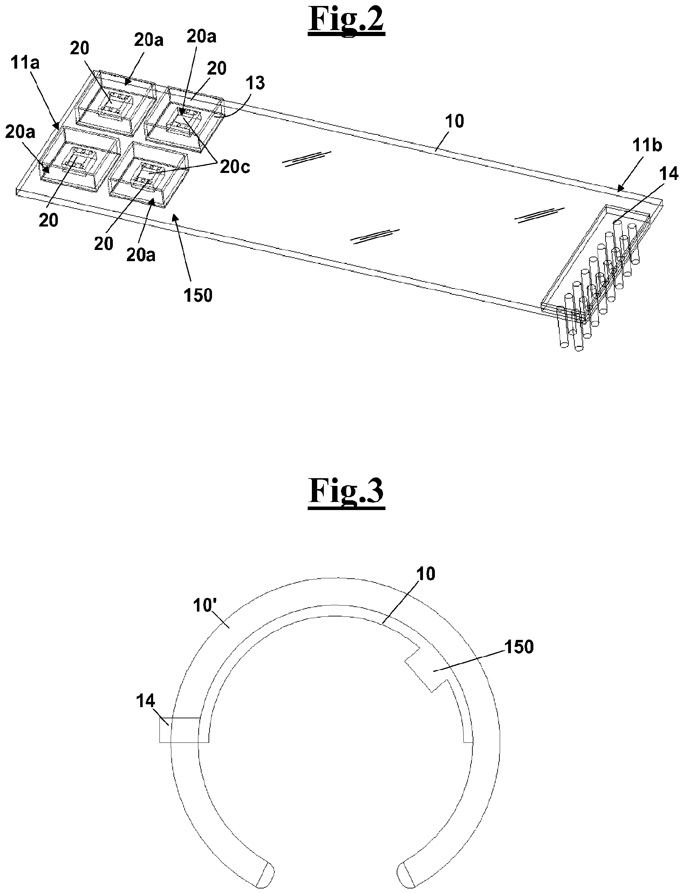

Detection unit 150 comprises a plurality, or an array, of pioezoresistive pressure sensors 20 (see for example Figs. 2,5,11).

More in detail, as diagrammatically shown in the block diagram of Fig. 1B, piezoresistive sensors 20 are arranged to provide a plurality of measurement signals 25 when detection unit 150 detects the pressure change within artery path 52 of patient 50. This circumstance, as described in detail hereinafter, takes place when artery 52 receives a predetermined force suitable for causing a predetermined flattening of the artery.

Tonometer 100 also comprises a control unit 30, as diagrammatically shown, that is associated with the plurality of pressure sensors 20 and is arranged to receive the plurality of measurement signals 25, each of which is associated with a respective sphygmic wave that has a determined amplitude. For example, Fig. 1A relates to the sphygmic waves of four measurement signals 25a-25d for each of which the respective amplitude A1-A4 is indicated.

Control unit 30 comprises a recognition means 32 configured to identify a reference signal 26 associated with a corresponding reference sensor 21 of the array of sensors 20, among the plurality of detected measurement signals 25, by means of a given recognition algorithm. More in detail, reference signal 26 corresponds to measurement signal 25 of the above-mentioned plurality, where said reference signal is associated with the sphygmic wave that has the largest amplitude.

In particular, reference sensor 21 that is associated with reference signal 26 is the one, among the plurality of sensors 20, which is arranged most proximate to artery path 52, with respect to the remaining part of sensors.

The array of sensors 20 and the detection at different times of reference sensor 21 assists the patient to wear support body 10 and to position respective detection unit 150 approximately upon the artery.

In particular, unlike the tonometric devices of known type, the assistance is not required of medical or paramedical staff, i.e. of a person specialized for correctly positioning a single sensor on artery 52. This allows tonometer 100, according to the present invention, to be used also directly by the end user at home, and therefore without requiring a nurse to position it every time. Furthermore, if wearable support 10, and in particular detection unit 150, can be displaced, for example, due to the rotation of the support about the user's wrist, the recognition procedure of reference sensor 21 is repeated at a plurality of instants (t₁, t₂, t₃,..., tₙ) . This way, the measure is independent from the movement of detection unit 150, since an optimum reference signal from which reference signal 26 is identified.

More in particular, recognition means 32 is configured to process the frequency spectrum of measurement signals 25 in a predetermined frequency band to which the signal pressure belongs, for example a cardiac frequency belongs to said frequency band.

Moreover, as shown always in Fig. 1B, recognition means 32 is configured to recognize, in the remainder part of the array of sensors 20, at least one boundary signal 27 with respect to reference signal 26. More in detail, boundary signal 27 is associated with a respective boundary sensor 22 and corresponds to measurement signal 25 of the above-mentioned plurality, which is associated with a sphygmic wave that has the smallest amplitude.

Moreover, control unit 30 comprises a means 34 for actuating (t₁, t₂, t₃, ..., tₙ) the recognition means at predetermined instants, in order to measure (t₁, t₂, t₃,..., tₙ) a reference measurement signal 26 and a relative reference sensor 21 at each instant. This way, it is always possible to determine sensor 20 that is arranged most proximate to artery path 52 among all the sensors 20 of the above-mentioned plurality. In particular, the step of identifying sensor 20 is possible even in the case of an accidental movement of detection unit 150 with respect to artery path 52.

Piezoresistive sensors 20 are arranged to recognize both a "useful" reference signal and a noise signal, which comprises the artifact signal caused by the movement, and the "hold-on pressure" signal, i.e. a signal related to the pressure that is required to flatten artery 52. More in detail, the sensor most proximate to the artery detects both the useful signal and the noise signal, whereas the boundary sensor 22 that is arranged at a longer distance from artery 52 than reference sensor 21, detects the noise signal only. This way, it is possible to precisely identify the useful signal by processing the data measured by reference sensor 21 and by subtracting the data measured by the boundary sensor 22 from it.

Therefore, the use of piezoresistive sensors 20 makes it possible to process more easily the data measured by the sensor elements, than in the prior art solutions, e.g. in the case of two-dimensional arrays of optical sensors, while obtaining more precise and more reliable results.

Control unit 30 comprises a program means 35 that is configured to process boundary signal 27 by a filtering algorithm 38, in order to extract an additional reference signal 26', i.e. the sphygmic component of boundary signal 27, and/or an artifact signal 28, i.e. the noise component of boundary signal 27.

According to a first exemplary embodiment of the present invention, as diagrammatically shown in Fig. 1B, program means 35 is adapted to combine additional reference signal 26' with reference signal 26 by a merging algorithm 36. This way, an enhanced reference signal 26" is obtained that has an increased information and quality with respect to reference signal 26. Enhanced reference signal 26" is then processed by program means 35 by a transformation algorithm 39, to provide a patient's arterial pressure real-time value 40'.

As diagrammatically shown in Fig. 1C, as an alternative to what described with reference to Fig. 1B, control unit 30 may extract from boundary signal 27 an artifact signal 28.

In this case, program means 35 is adapted to subtract artifact signal 28 from reference signal 26, by a subtraction algorithm 37, thus obtaining a correct reference signal 26"', i.e. without the artifact component 28. Correct reference signal 26''' is then processed by program means 35 by a transformation algorithm 39, to provide a patient's arterial pressure real-time value 40.

In the further exemplary embodiment, as diagrammatically shown in Fig. 1D, once enhanced reference signal 26" has been obtained, as described in Fig. 1B, program means 35 carries out the subtraction algorithm 37 to subtract artifact 28 from the enhanced signal 26". In this case, program means 35 uses therefore both the algorithms, i.e. union and differential algorithms.

This way, an improved and correct reference signal 26* is obtained that, besides having an improved information content with respect to reference signal 26, does not contain artifact 28 and is therefore a signal of very high quality. Enhanced and correct reference signal 26* is then processed by transformation algorithm 39 to provide a real-time value of patient's arterial pressure 40*.

While processing the data, besides eliminating artifacts 28, control unit 30 can eliminate possible hold-on pressure variations with respect to the value that has been determined in a preliminary calibration step. The latter may conventionally measure the patient's maximum pressure, minimum pressure and mean pressure, by means of a sleeve, i.e. a cuff, and may associate the measured pressure values to the above defined correct and/or improved signal.

Even if reference has been made above to a boundary signal 27 that is computed by program means 35 to improve the quality of reference measurement signal 26, obtaining an improved and/or correct reference signal, program means 35 can also use all measurement signals 25, apart from reference signal 26, as boundary signals. In this case, all measurement signals 25, and therefore all sensors 20, are used both to improve reference signal 26 and to find out artifact signal 28.

In other words, all measurement signals 25 take part both to the artifact removal step, by the subtraction algorithm, and to the step of the information content improvement signal 26 by the merging algorithm, thanks to the increased available data flow. In other words, all sensors 20 of the array are useful not only to reduce the noise, but also to increase the quality of the signal.

In particular, two signals are identified, i.e. a signal s(t) and a noise n(t). Each sensor detects, in particular, a linear combination of the two signals. Accordingly, once accepted the two sources model (signal and noise) that can be considered uncorrelated and even independent from each other, it is possible to divide the two sources by blind decomposition methods, such as the Principal Component Analysis (PCA), or the Independent Component Analysis (ICA).

In particular, PCA is based on the signal covariance matrix, and calculates its eigenvalues. In our case, 4 signals = 4 eigenvalues. Still theoretically, the 2 highest eigenvalues should "define" large part of the variance (i.e. of the information content) of the signals. As an alternative, an algorithm can be provided that analyses the spectrum of the combined signal comprising the signal and the noise, and are able to separate the signal from the noise, since some features of the signal of interest are known, such as the frequency band to which the pressure signal belongs (about the cardiac frequency).

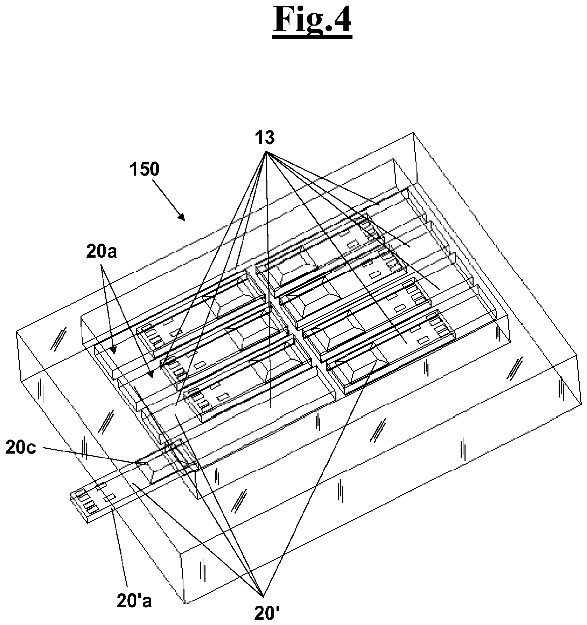

From a structural viewpoint, as shown in Fig. 2 or 4, detection unit 150 comprises a plurality of housings 13, each of which is arranged to house a respective sensor 20, in order to form a sensor unit 20a with it.

In particular, in a first exemplary embodiment, each housing is made of a flexible material, in particular it is made of a silicone rubber. This allows transmitting the external forces to the sensor that is contained within the housing.

As an alternative, each housing 13 is made of a stiff material, for example a ceramic material. Then, each housing 13A receives a fluid material, in particular a silicone rubber which, once polymerized, transmits the external forces to sensor 20 that is contained within stiff housing 13. In particular, each stiff housing 13 houses a sensor that comprises only a sensitive element 20c. As well known, the piezoresistive sensitive element 20c generally comprises a membrane that changes its shape responsive to the pressure variation that is transmitted by artery path 52, with which it comes into contact.

In this case, sensor units 20a are arranged at a predetermined distance, in order to define the array of sensors 20.

In particular, detection unit 150 comprises several sensor elements 20a according to a predetermined layout, for example 2x2,2x4, or normally 2xN, where N represents the number of sensor elements 20a.

Sensor elements 20a are located on support body 10, in particular on a flexible support body that is configured to be arranged about the user's wrist 50, as diagrammatically shown in Fig. 3. In particular, sensor elements 20a are arranged at an end portion 11a of flexible support body 10. At a second end 11b, opposite to first end 11a, support body 10 comprises a feed and output connector 14 of sensors 20. This way, by positioning connector 14 on the side opposite to sensors 20, the operation of the tonometer is assisted, since the sensitive part, in use, is arranged on the wrist. Furthermore, this connector 14 is located in a position that is, in use, at a distance of several cm from the array, so that the bending of the flexible support is not hindered by the presence of the connector itself.

In particular, flexible support body 10 may comprise, for instance, a stiff bracelet ring element 10', that has a recess to provide an access to connector 14 of the support of the sensors, on which it is connected, in order to maintain detection unit 20 oriented towards the artery path of patient 50, and in order to keep its position stable enough to avoid any nuisance to the patient. Such an embodiment is particularly easy and comfortable to use and to wear. This allows using it like a simple wristwatch that, accordingly, can be worn permanently, to detect measurement signals obtained from pressure sensors 20.

In the exemplary embodiment of Fig. 4, housings 13 have a box-like elongated shape and are adapted to house an elongated sensor 20'. In this case, sensor units 20a are arranged adjacent to one another, in order to define the array of sensors 20.

More in detail, each elongated sensor 20' comprises a support base 20'a on which a sensitive element 20c is arranged and is connected to support base 20'a.

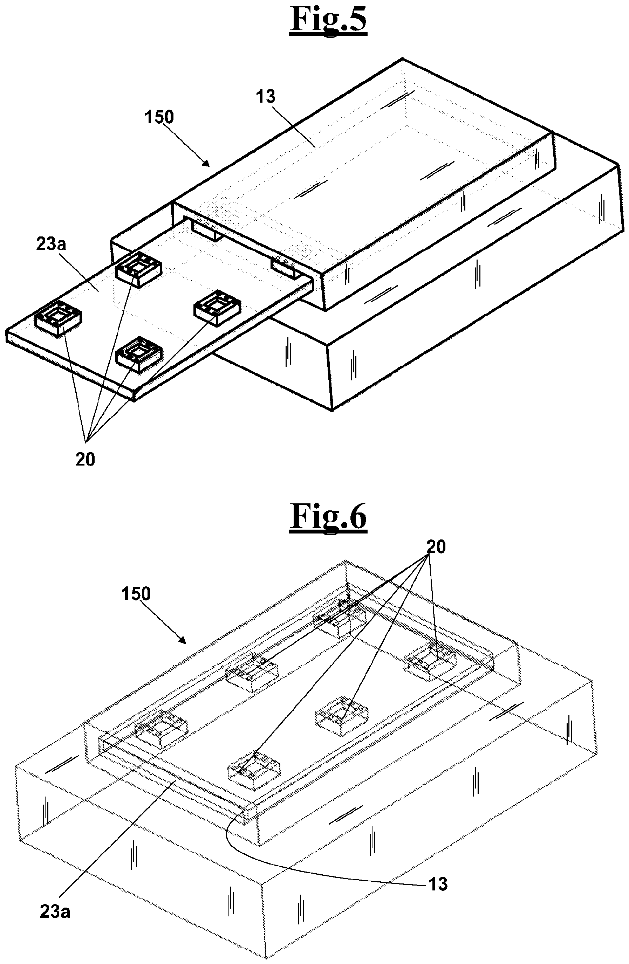

As an alternative, as shown in Figs. 5 and 6, detection unit 150 comprises a single housing 13 that is arranged to house a sensorized sheet 23a on which the plurality of sensors 20 is arranged.

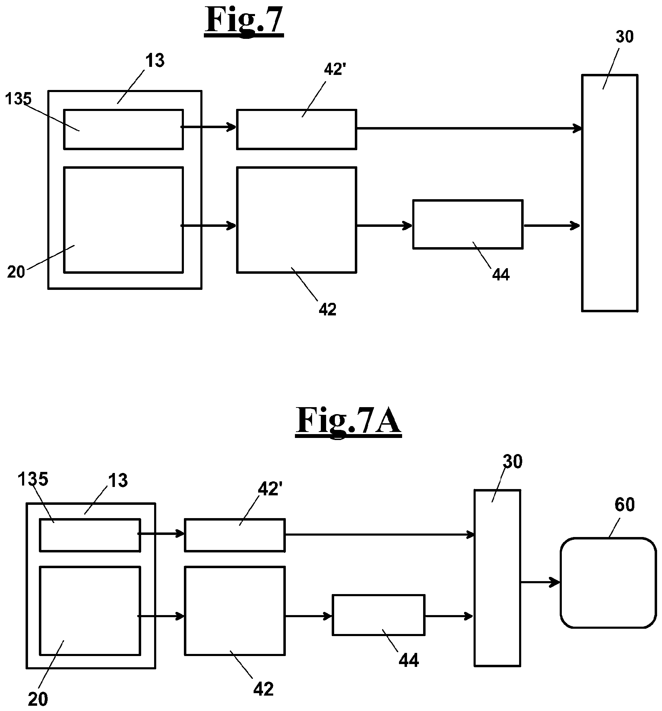

As diagrammatically shown in Fig. 7, the packaging 13 of box-like portion 12 contains the array of piezoresistive sensors 20 and also contains a temperature sensor 135 configured to compensate the temperature drift which, as well known, is likely to take place in piezoresistive sensors 20. Packaging 13 is connected to control unit 30 as described hereinafter. Between sensors 20 and control unit 30 an analog front-end, or AFE, 42 may be provided, and an analog - digital converter 44. Also temperature sensor 135 may be connected to control unit 30, by a respective Analog front-end, or AFE, 42'. Control unit 30 compensates for the temperature drift of sensors 20 on the basis of the measurements made through temperature sensor 135, and analyses the measurement signals 25 detected by sensors 20, as described with reference to Figs. 1A to 1D.

As shown, for instance, in Fig. 7A, a display interface 60 may be provided on wearable support body 10, in particular a display unit may be provided, which is arranged, in use, opposite to detection unit 150.

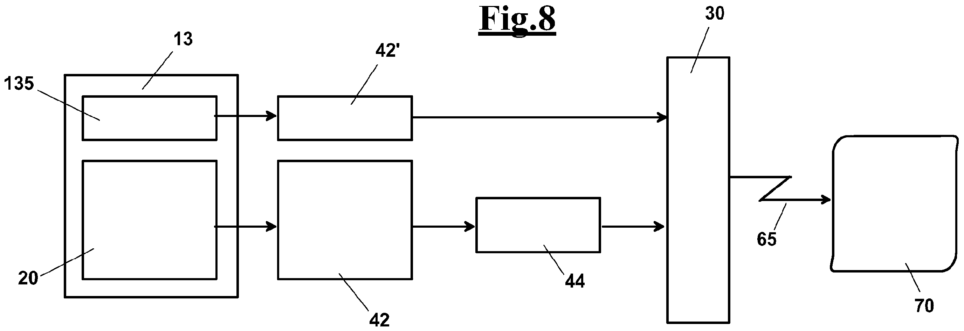

As shown in Fig. 8, control unit 30 also comprises a transmission means 65, in particular a wireless means, that is configured to transmit the data measured by the plurality of sensors 20 to a remote unit 70. As an alternative, control unit 30 may comprise a memory unit configured to memorize the plurality of measurement signals 25 and to successively transfer them to remote unit 70.

Besides pressure sensors 20, wearable support body 10 may comprise further sensors in order to improve the available data, for instance, the further sensors are an accelerometer configured to eliminate the artifacts due to the movement of patient 50, a biochemical sensor configured to measure the electrolytes in the skin, to increase physiological or pathological data of patient 50, a temperature sensor 135, to compensate the intrinsic reading changes of the piezoresistive sensors.

Moreover, in order to assist positioning of tonometer 100, an actuator and a control system may be provided to obtain an optimum positioning at artery path 52. In particular, the actuator is adapted to apply a flattening pressure that is most suitable for detecting the sphygmic wave on artery path 52.

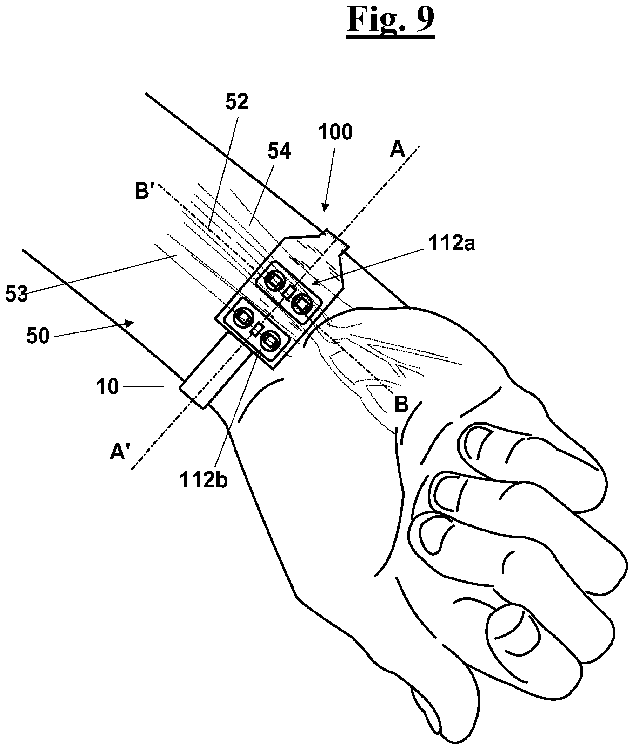

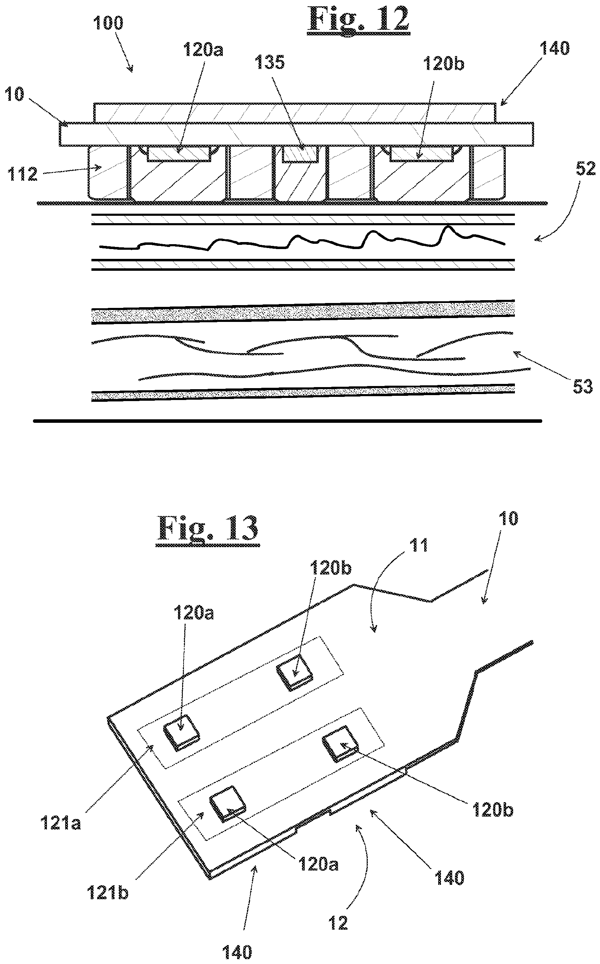

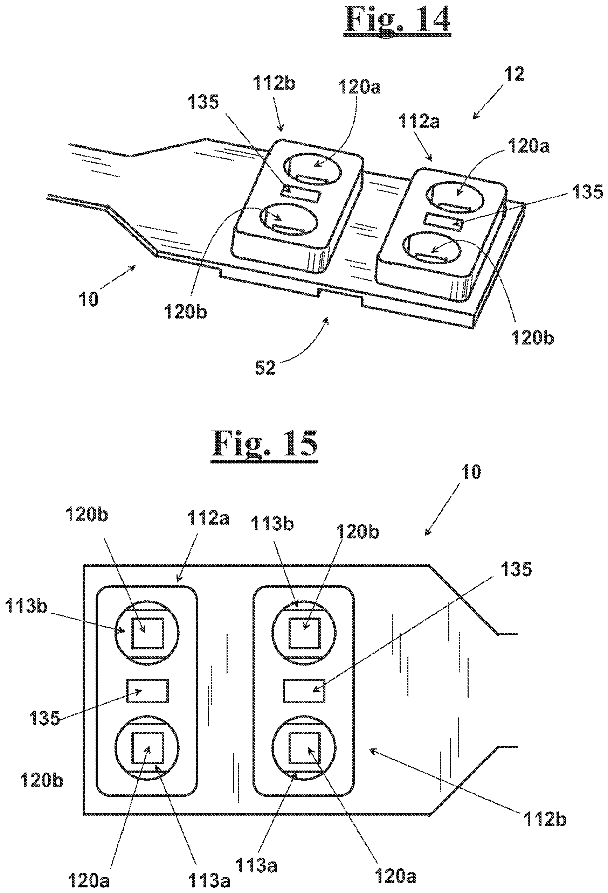

In the exemplary embodiment diagrammatically shown in Figs. 9 to 15, tonometer device 100 comprises a wearable support body 10 made of a flexible material, in particular of flexible Printed Circuit Boards, e.g. PCB flex, which has a first side 11 and a second side 12 opposite to first side 11.

A predetermined number of piezoresistive sensor elements 120 is arranged to form rows 121 on first side 11 of wearable support body 10, said elements connected to the first side of the base layer by means of a "wirebond" technique.

More in detail, in the exemplary embodiment of Figs. 9 to 15, tonometer 100 provides a main body 125 of a stiff material that is connected to first side 11 of wearable support body 10, for example by gluing, and has a first and a second opening 113a and 113b. At each opening 113a and 113b is arranged, in use, a respective piezoresistive sensitive element 120a,120b of a predetermined row of piezoresistive sensor elements, or "die", i.e. the essential part of a piezoresistive sensor.

Each opening 113a and 113b is adapted to be filled with a predetermined amount of fluid silicone rubber in order to fully cover the respective piezoresistive sensitive element 120a,120b that is housed within this opening. Once hardened, the silicone rubber provides a transmission element 160 that protrudes from main body 125 and is adapted to transmit the external forces to piezoresistive sensitive element 120.

In particular, main body 125 serves to protect the couple of sensors from dirt, powder and shocks and to stiffen the overall structure along a determined direction as explained in detail hereinafter. Main body 125 also serves for covering sensitive element 120 with fluid silicone rubber through openings 133.

The group consisting of a main body 125, a force transmission element, and a row of piezoresistive sensor elements 120a,120b, forms a sensor unit 112. Therefore, in the case diagrammatically shown in Figs. 9 to 15, a first sensor unit 112a and a second sensor unit 112b are provided.

According to the invention, each main body 125 may also comprise a third opening 133c, which may be located between two adjacent piezoresistive sensor elements 120a and 120b of the row. A temperature sensor 135 is arranged at opening 133c. As well known, piezoresistive sensors 120 cold be affected by a considerable temperature drift if the temperature changes. Therefore, temperature sensor 135 serve to record possible temperature changes, in order to compensate the above temperature drift. In order to increase the patient's comfort, and to arrange temperature sensor 135 at a same condition as piezoresistive sensors 20, a further silicone rubber or rubber layer may be provided on the transmission element, or a layer made of any biocompatible material.

Besides, tonometer 100 can also provide a stiffening element 140 that is arranged on the second side of the flexible base support. More in detail, a stiffening element 140 may be provided, for example a stiffening element made of a plastic stiff material such as a stiff PCB, which is arranged on the second side at a respective main body 125, i.e. at a mirror-like position opposed to it, with respect to the flexible base support.

This way, an object is obtained that is considerably stiff at stiffening bodies 140 and at main bodies 125, in order to ensure, among other things, the integrity of the welded joints made with the "wire bond" technique, and with a remarkable flexibility, between two adjacent main bodies 125 thanks to support body 10 made of a flexible material that assists arranging device 100 about the user's wrist. More in detail, tonometer device 100 is positioned on user's wrist, in such a way to be very stiff along the longitudinal direction of artery 52 and to be flexible along the transversal direction.

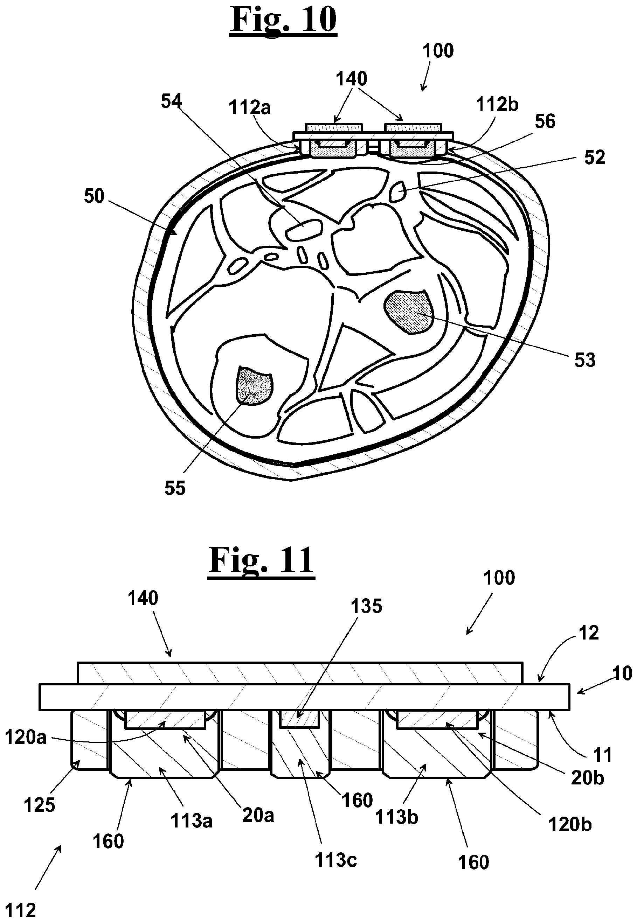

The above described particular technical solution for tonometer device 100, according to the invention, which comprises at least one first and a second sensor units 112a and 112b, each of which is equipped with a respective row 121a,121b of sensor elements 120 comprising at least one first and at least one second sensitive elements 120a and 120b, has different advantages. Firstly, this layout makes it possible to arrange a row of sensor elements 120 in a position well suited for detecting the arterial pressure, i.e. in the case diagrammatically shown in Fig. 10, at a recess 56 between the radial bone 53 and a tendon 54. Once sensor unit 112 that is arranged in the above correct position has been identified, a search begins among all the sensors of the row of this sensor unit 112, of sensitive element 120a or 120b that can detect the best measurement signal. In this connection, it should be remarked that the quality of the signal changes responsive to the proximal distance from the artery.

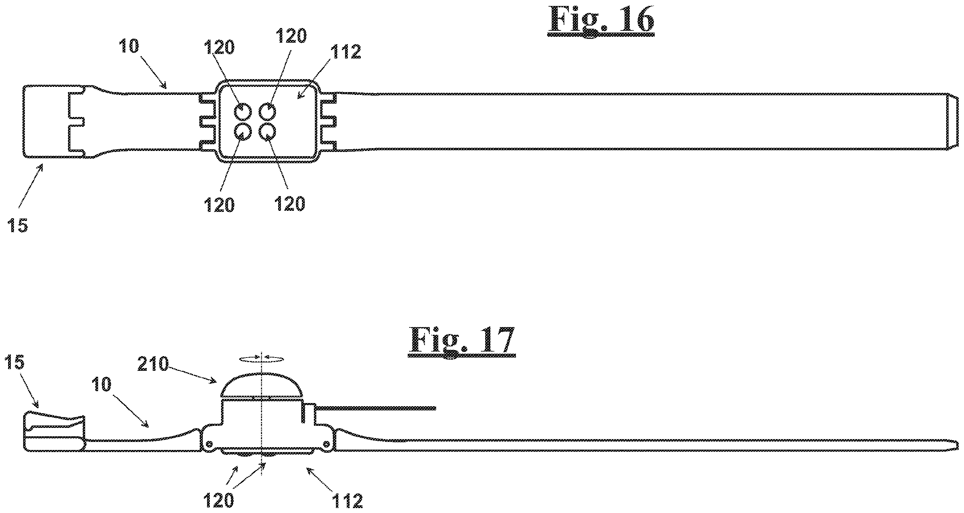

In the exemplary embodiment diagrammatically shown in Figs. 16 to 19, a distance adjustment means 210 is also provided for adjusting the distance of sensor unit 112 from the artery path of patient 50 52. More in detail, adjustment means 210 is adapted to remove/approach sensor unit 112 from/to artery path 52, in order to flatten it accordingly. As well known, a pressure sensor signal can detect the arterial pressure only if artery 52 is slightly flattened. More in detail, a lower limit value exists of the flattening of artery 52, below which the pressure wave cannot be measured, as well as an upper limit of the flattening of artery 52, above which the artery itself is blocked. Therefore, the means for adjusting distance 210 of sensor unit 112 from artery path 52 serves for applying a pressure on the patient's wrist that is strong enough to flatten artery 52 and, accordingly, to allow detecting the pressure signal by sensor unit 112, but not so strong to block artery 52 itself. Advantageously, adjustment means 210 may a manual means, to be manually operated by the patient himself.

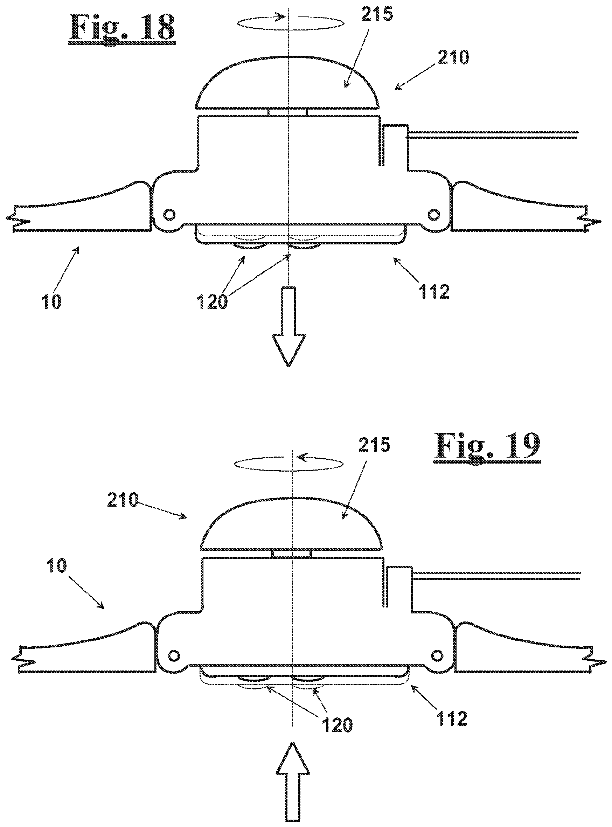

For example, adjustment means 210 may comprise a worm screw, not shown in detail in Fig., which has an end portion integral to a knob 215, or to a different interaction element. When positioning sensor unit 112 with respect to artery 52, patient 50 can act manually on knob 215 in order to slide sensor unit 112 towards/away from artery 52. More in detail, if the knob is turned in a first rotation direction, sensor unit 112 is moved towards artery 52, in order to flatten the latter (Fig. 18), whereas if knob 215 is turned in the opposite direction, sensor unit 112 is moved in the direction opposite to the previous direction, i.e. sensor unit 112 is removed from artery 52 (Fig. 19).

Wearable body 10 may also comprise a closure 15 that allows a steady lock about patient 50's wrist and then allows of keeping sensor unit 112 in the correct position with respect to artery 52.

Furthermore, a control means, not shown in the figures, can be provided on board of tonometer structure 100, which is configured to read the pressure signal detected by sensor unit 112, and configured to compare it with a reference value. More in detail, when value of the detected pressure signal is higher than the reference value, a display and/or a sound emitter of the tonometer communicates to the patient that the detected signal is strong enough for detecting the arterial pressure.

## Claims

1. A tonometer structure (100) for continuously monitoring a patient's arterial pressure for a determined time, which tonometer structure (100) comprises:
- a wearable support body (10) arranged to be worn by a patient (50);
- a detection unit (150) mounted to said wearable support body (10), said support body (10) and said detection unit (150) configured in such a way that said detection unit, in use, contacts said patient's skin (50) proximate to an artery path (52), said detection unit (150) having a plurality of piezoresistive pressure sensors (20), each sensor of said plurality configured in such a way to measure a pressure variation when said detection unit (150) contacts said patient's skin, and configured to provide a respective measurement signal (25), said measurement signal (25) associated with a respective sphygmic wave of a determined amplitude wherein said tonometer structure (100) further comprises:
- a control unit (30) associated with said plurality of pressure sensors (20) and arranged to receive said plurality of measurement signals (25), said control unit equipped with a recognition means (32) being programmed with a recognition algorithm configured to process said plurality of measurement signals and comprising the steps of:
- recognizing a reference signal (26) among said plurality of measurement signals (25), wherein said reference signal is associated with a respective reference sensor (21), wherein said reference signal is the measurement signal of said plurality associated with the sphygmic wave having the largest amplitude;
- recognizing one boundary signal (27) in the remaining part of said plurality of measurement signals (25), said one boundary signal associated with a respective boundary sensor (22), wherein said boundary signal (27) is the measurement signal (25) of said plurality associated with the sphygmic wave having the smallest amplitude;
- an actuation means (34) arranged to actuate said recognition means (32) at predetermined instants (t₁, t₂, t₃, tn), said recognition means (32) configured to measure a reference measurement signal (26) and to identify a relative reference sensor (21) at each of said instants (t₁, t₂, t₃, tn), such that also in the case of an accidental movement of said detection unit (150) with respect to said artery path (52), a reference sensor (21) is always recognized;
said control unit (30) also comprising a program means (35) including:
- a filtering algorithm configured to carry out a step of processing said boundary signal (27) for extracting from said boundary signal (27) a sphygmic component, and a noise component;
- a subtraction algorithm configured to carry out a step of subtracting said noise component of said boundary signal (27) from said reference signal to obtain a corrected reference signal that is depurated from artifacts and background noise;
- a transformation algorithm configured to carry out a step of processing said corrected reference signal to provide a real-time value (40, 40', 40*) of the patient's arterial pressure.

2. A tonometer structure (100) according to claim 1, wherein said program means is configured to carry out also the following steps:
- combining, by a merging algorithm, said reference signal and said sphygmic component of said boundary signal obtaining an enhanced reference signal, in order to increase the information and the quality of said reference signal itself;
- processing said enhanced reference signal by said transformation algorithm.

3. A tonometer structure (100) according to claim 1, wherein said recognition means is configured to process the frequency spectrum of said measurement signals (25) in a predetermined frequency band to which the signal pressure belongs, said predetermined frequency band belonging to a frequency band comprising said cardiac frequency.

4. A tonometer structure (100) according to claim 1, wherein said recognition means (32) is configured to recognize a plurality of boundary signals (27) with respect to said reference signal (26) in the remainder part of said plurality of sensors (20), said recognition means provided by said program means (35) in order to improve the information of said reference signal (26), by a merging algorithm (36), and in order to determine said artifact signal (28) to be subtracted from said reference signal (26) by a subtraction algorithm (37).

5. A tonometer structure (100) according to claim 1, wherein said detection unit (150) comprises a plurality of housings (13), each housing of said plurality of housings arranged to house a respective sensor (20), such that a sensor unit is formed (20a); in particular each housing (13) being made of a flexible material that is adapted to transmit the external forces to the sensor that is housed within said housing (13).

6. A tonometer structure (100) according to claim 5, wherein each housing (13) has a box-like elongated shape and is adapted to house an elongated sensor, said elongated sensor comprising a support base (20'a) and a sensitive element (20c) connected to said support base (20' a) .

7. A tonometer structure (100) according to claim 5, wherein said flexible material is silicone rubber each housing (13) being made of a stiff material and is arranged to be filled with a fluid polymeric material that, once hardened, is adapted to transmit the external forces to the sensor (20) contained in said housing made of a stiff material, each housing (13) made of a stiff material arranged to house a sensor that is formed by said sensitive element (20c) only.

8. A tonometer structure (100) according to claim 7, wherein said stiff material is a ceramic material.

9. A tonometer structure (100) according to claim 7, wherein said fluid polymeric material is silicone rubber.

10. A tonometer structure (100) according to claim 5, wherein said plurality of sensor units (20a) is arranged according to a predetermined layout (2xN), wherein 2xN is the number of said sensor elements (20a), on said support body, said sensor unit (20a) arranged at one end (11a) of said flexible support body (10) and provided with a feed connector (14) arranged at a second end (11b) opposite to said first end (11a).

11. A tonometer structure (100) according to claim 6, wherein said box-like shaped detection unit (150) comprises a single housing (13) arranged to house a sensorized sheet (13a) at which said plurality of sensors (20) is arranged.

12. A tonometer structure (100) according to claim 1, wherein said wearable support body (10) comprises, besides said pressure sensors (20), at least one auxiliary sensor configured to provide further useful data, said or each auxiliary sensor selected from the group consisting of:
- an accelerometer for eliminating the artifacts due to the patient's movement (50);
- a biochemical sensor for measuring electrolytes in the skin, for increasing the patient's (50) physiological or diagnostic data;
- a temperature sensor for compensating the intrinsic reading changes of the piezoresistive sensors;
- an actuator and a control device of said actuator for more easily positioning and attaining an optimum positioning at said artery path (52), said actuator arranged to apply to said artery path (52) an flattening pressure best suited for detecting the sphygmic wave;
- or a combination thereof; in particular comprising a base body provided with said or each further sensor, said base body arranged to be removably connected to said tonometer structure in order to allow providing said further sensors.

13. A tonometer structure (100) according to claim 1, comprising:
- a wearable support body (10) made of a flexible material, said wearable support body (10) having a first side (11) that, in use, can be oriented towards the user's skin (50) and a second side (12) opposite to the first side;
- a plurality of piezoresistive elements (120) arranged to form rows (121), each piezoresistive element (120) of said plurality arranged at said first side (11) of said wearable support body (10);
- a main body made of a stiff material (125) arranged at said first side (11) of said wearable support body (10), said main body (10) having a plurality of openings (113) at each of which, in use, a respective piezoresistive sensitive element (120) is arranged of one of said rows, such that a sensor unit (112) is formed;
- each opening (113) of said plurality arranged to be filled with a predetermined amount of a fluid polymeric material in order to fully cover the respective piezoresistive sensitive element (120) that is housed within said opening, said fluid polymeric material adapted to harden such that a transmission element (160) is provided arranged to transmit the external forces to the piezoresistive sensitive element (120), which protrudes from said main body (125), in particular said fluid polymeric material is silicone rubber; in particular said flexible material being a flexible Printed Circuit Boards, in particular a PCB flex; in particular each main body being also provided with a third opening, said third opening arranged, in use, to house a temperature sensor.

14. A tonometer structure (100) according to claim 13, wherein a further silicone rubber or rubber layer is provided on said transmission element, or a layer is provided made in any biocompatible material that is adapted, in use, to be brought into contact of the user's skin, in order to increase the patient's comfort.

15. A tonometer structure (100) according to claim 13, wherein a stiffening element is also provided, in particular a stiffening element made of a plastic stiff material such as a stiff PCB, which is arranged on said second side of said wearable support body at a respective main body, i.e. at a mirror-like position opposed to said main body, with respect to said wearable support body ,said stiffening bodies and said main bodies being arranged, in use, along an axial direction of said artery path; in particular an adjustment means being also provided for adjusting the distance of said sensor unit from said artificial artery, said adjustment means arranged to remove/approach said sensor unit from/to said artery path, to cause said artery path to become flat, in particular a control means being provided configured to read said detected pressure signal and to compare it with a reference value such that, when said value of said detected pressure signal is higher than said reference value, a display and/or a sound emitter of said tonometer communicates to the patient that the detected signal is strong enough for detecting the arterial pressure.

## Patentansprüche

1. Eine Tonometerstruktur (100) zur kontinuierlichen Überwachung des arteriellen Druckes eines Patienten für eine vorgegebene Zeit, wobei die Tonometerstruktur (100) umfasst:
- ein tragbares Stützeil (10), welches dazu ausgelegt ist, von einem Patienten (50) getragen zu werden;
- eine Detektionseinheit (150), die an dem besagten tragbaren Stützteil (10) montiert ist, wobei das besagte Stützteil (10) und die besagte Detektionseinheit (150) derart ausgelegt sind, dass die besagte Detektionseinheit (150) beim Gebrauch die Haut des besagten Patienten (50) in der Nähe einer Arterienzweigs (52) berührt, wobei die besagte Detektionseinheit (150) eine Mehrzahl von piezoresistiven Drucksensoren (20) aufweist, wobei jeder Sensor dieser besagten Mehrzahl dazu ausgelegt ist, eine Druckveränderung zu messen, wenn die Detektionseinheit (150) die Haut des Patienten berührt, sowie dazu, ein entsprechendes Messsignal (25) zu liefern, wobei das besagte Messsignal (25) einer entsprechenden sphygmischen Welle einer bestimmten Amplitude zugeordnet ist,
wobei die Tonometerstruktur (100) ferner umfasst:
- eine Steuereinheit (30), die der besagten Mehrzahl von Drucksensoren (20) zugeordnet sowie derart ausgelegt ist, um die besagte Mehrzahl von Messsignalen (25) zu empfangen, wobei die besagte Kontrolleinheit mit einem Erkennungsmittel (32) ausgestattet ist, das mit einem Erkennungsalgorithmus programmiert ist, der dazu ausgelegt ist, die besagte Mehrzahl von Messsignalen zu verarbeiten, und folgende Schritte umfasst:
- Erkennen eines Referenzsignals (26) unter der besagten Mehrzahl von Messsignalen (25), wobei das besagte Referenzsignal einem entsprechenden Referenzsensor (21) zugeordnet ist, wobei das besagte Referenzsignal dasjenige Messsignal aus der besagten, der sphygmischen Welle zugeordneten Mehrzahl ist, welches die größte Amplitude aufweist;
- Erkennen eines Grenzwertsignals (27) in dem verbleibenden Rest der Mehrzahl von Messsignalen (25), wobei das besagte Grenzwertsignal einem entsprechenden Grenzwertsensor (22) zugeordnet ist, wobei das besagte Grenzwertsignal (27) dasjenige Messsignal (25) aus der besagten, der sphygmischen Welle zugeordneten Mehrzahl ist, welches die kleinste Amplitude aufweist;
- ein Aktivierungsmittel (34), das zum Aktivieren des besagten Erkennungsmittels (32) zu festgelegten Augenblicken (t1,t2,t3,tn) eingerichtet ist, wobei das Erkennungsmittel (32) dazu ausgelegt ist, zu jedem besagten Augenblick (t1,t2,t3,tn) ein Referenzsignal (26) zu messen und einen jeweiligen Referenzsensor (21) zu identifizieren, so dass auch im Falle einer unbeabsichtigten Bewegung der besagten Detektionseinheit (150) gegenüber dem besagten Arterienzweig (52) ein Referenzsensor (21) immer erkannt wird;
wobei die besagte Steuereinheit (30) auch ein Programmmittel (35) aufweist, umfassend:
- einen Filteralgorithmus, der dazu ausgelegt ist, einen Schritt der Verarbeitung des besagten Grenzwertsignals (27) durchzuführen, um eine sphygmische Komponente und eine Rausch-Komponente aus dem besagten Grenzwertsignal (27) zu extrahieren,
- einen Subtraktionsalgorithmus, der dazu ausgelegt ist, einen Schritt auszuführen der Subtraktion der besagten Rausch-Komponente des Grenzwertsignals (27) von dem Referenzsignal, um ein korrigiertes Referenzsignal zu erhalten, das von den Artefakten und vom Hintergrundrauschen bereinigt ist;
- einen Transformationsalgorithmus, der dazu ausgelegt ist, einen Schritt der Verarbeitung des besagten korrigieren Referenzsignals auszuführen, um einen Echtzeitwert (40,40',40*) von dem arteriellen Druck des Patienten zu erhalten.

2. Eine Tonometerstruktur (100) gemäß Anspruch 1, wobei das besagte Programmmittel derart konfiguriert ist, auch folgende Schritte auszuführen:
- Kombinieren des besagten Referenzsignals mit der sphygmischen Komponente des besagten Grenzwertsignals durch einen Verschmelzungsagorithmus, wobei ein verstärktes Referenzsignal erhalten wird, um die Information und die Qualität des besagten Referenzsignals selbst zu steigern;
- Verarbeiten des besagten verstärkten Referenzsignals mittels des besagten Transformationsalgorithmus.

3. Eine Tonometerstruktur (100) gemäß Anspruch 1, wobei das Erkennungseinmittel dazu ausgelegt ist, das Frequenzspektrum des besagten Messsignals (25) in einem vorbestimmten Frequenzband, zu welchem der Signaldruck gehört, zu verarbeiten, wobei das besagte vorbestimmte Frequenzband zu einem Frequenzband gehört, das die besagte Herzfrequenz enthält.

4. Eine Tonometerstruktur (100) gemäß Anspruch 1, wobei das Erkennungsmittel (32) dazu ausgelegt ist, in dem restlichen Teil der besagten Mehrzahl von Sensoren (20) eine Mehrzahl von Grenzwertsignalen (27) bezüglich des besagten Referenzsignals (26) zu erkennen, wobei das besagte Erkennungsmittel von dem Programmmittel (35) bereitgestellt wird, um die Information des besagten Referenzsignals (26) durch einen Verschmelzungsalgorithmus (36) zu verbessern, und um das besagte Artefaktsignal (28) zu bestimmen, das von dem besagten Referenzsignal (26) mittels eines Subtraktionsalgorithmus (37) zu subtrahieren ist.

5. Eine Tonometerstruktur (100) gemäß Anspruch 1, wobei die Detektionseinheit (150) eine Mehrzahl von Gehäusen (13) umfasst, wobei jedes Gehäuse der Mehrzahl von Gehäusen dazu ausgelegt ist, einen jeweiligen Sensor (20) zu beherbergen, so dass eine Sensoreinheit (20a) gebildet wird; insbesondere besteht jedes Gehäuse (13) aus einem elastischen Material, welches dazu geeignet ist, die externen Kräfte zu dem Sensor zu übertragen, der in dem besagten Gehäuse (13) untergebracht ist.

6. Eine Tonometerstruktur (100) gemäß Anspruch 5, wobei jedes Gehäuse (13) eine schachtelförmige längliche Gestalt hat und dazu geeignet ist, einen länglichen Sensor aufzunehmen, wobei der besagte längliche Sensor eine tragende Basis (20'a) umfasst sowie ein sensitives Element (20c), das an der besagten tragenden Basis (20'a) angeschlossen ist.

7. Eine Tonometerstruktur (100) gemäß Anspruch 5, wobei das besagte elastische Material Silikonkautschuk ist, wobei jedes Gehäuse (13) aus einem steifen Material besteht und dafür eingerichtet ist, mit einem flüssigen Polymermaterial gefüllt zu werden, welches in ausgehärtetem Zustand in der Lage ist, die externen Kräfte zu dem Sensor (20) zu übertragen, der in dem besagten Gehäuse aus einem steifen Material enthalten ist, wobei jedes Gehäuse (13) aus einem steifen Material dazu ausgelegt ist, einen Sensor zu beherbergen, der einzig aus dem sensitiven Element (20c) besteht.

8. Eine Tonometerstruktur (100) gemäß Anspruch 7, wobei das besagte steife Material ein keramisches Material ist.

9. Eine Tonometerstruktur (100) gemäß Anspruch 7, wobei das besagte flüssige Polymermaterial Silikonkautschuk ist.

10. Eine Tonometerstruktur (100) gemäß Anspruch 5, wobei die besagte Mehrzahl von Sensoreinheiten (20a) auf dem besagten Stützteil (10) nach einem vorgegebenen Layout (2xN) angeordnet ist, wobei 2xN die Anzahl der besagten Sensorelemente (20a) ist, wobei die besagte Sensoreinheit (20a) an einem Ende (11a) des flexiblen Stützteils (10) angeordnet ist und mit einem Zuführanschluss (14) versehen ist, der an einem dem besagten ersten Ende (11a) gegenüber liegenden zweiten Ende (11b) angeordnet ist.

11. Eine Tonometerstruktur (100) gemäß Anspruch 6, wobei die schachtelförmig gestaltete Detektionseinheit (150) ein einzelnes Gehäuse (13) umfasst, welches derart angeordnet ist, um eine Sensor-Platte (13a) aufzunehmen, auf welcher die besagte Mehrzahl von Sensoren (20) angeordnet ist.

12. Eine Tonometerstruktur (100) gemäß Anspruch 1, wobei das besagte tragbare Stützteil (10) neben den besagten Drucksensoren (20) wenigstens einen Hilfssensor aufweist, der dazu ausgelegt ist, weitere hilfreiche Daten bereitzustellen, wobei der besagte oder jeder Hilfssensor ausgewählt ist aus der Gruppe bestehend aus:
- ein Beschleunigungsmesser, um die von der Bewegung des Patienten (50) herrührenden Artefakte zu eliminieren;
- einen biochemischen Sensor zur Messung der Elektrolyten in der Haut, um die physiologischen oder die diagnostischen Daten des Patienten (50) zu erweitern;
- einen Temperatursensor zum Ausgleichen der intrinsischen Erfassungsabweichungen der piezoresistiven Sensoren;
- einen Aktivator und eine Steuereinheit des besagten Aktivators, zum einfacheren Positionieren und um eine optimale Positionierung an dem besagten Arterienzweig (52) zu erhalten, wobei der besagte Aktivator derart angeordnet ist, um auf den besagten Arterienzweig (52) einen abflachenden Druck auszuüben, der am besten für die Detektion der sphygmischen Welle geeignet ist;
- oder eine Kombination davon; insbesondere umfassend einen Basiskörper, der mit dem besagten oder jedem weiteren Sensor versehen ist, wobei der besagte Basiskörper derart angeordnet ist, um lösbar an der besagten Tonometerstruktur angeschlossen zu werden, um eine Ausstattung mit den besagten weiteren Sensoren zu ermöglichen.

13. Eine Tonometerstruktur (100) gemäß Anspruch 1, umfassend:
- ein tragbares Stützteil (10) aus einem elastischen Material, wobei das besagte tragbare Stützteil (10) eine erste Seite (11) aufweist, die beim Gebrauch in Richtung zu der Haut des Anwenders (50) ausgerichtet werden kann, sowie eine der ersten Seite gegenüber liegende, zweite Seite (12);
- eine Mehrzahl von piezoresistiven Elementen (120), welche derart angeordnet sind, um Reihen (121) zu bilden, wobei jedes piezoresistive Element (120) der besagten Mehrzahl an der besagten ersten Seite (11) des tragbaren Stützteils (10) angeordnet ist;
- einen an der besagten ersten Seite (11) des tragbaren Stützteils (10) angeordneten Hauptkörper aus einem steifen Material (125), wobei der besagte Hauptkörper (10) eine Mehrzahl von Öffnungen (113) aufweist, wobei an jeder davon während des Gebrauchs ein entsprechendes piezoresistives sensitives Element (120) aus einer der besagten Reihen angeordnet ist, so dass eine Sensoreinheit (112) gebildet wird;
- wobei jede Öffnung (113) der besagten Mehrzahl derart angeordnet ist, um mit einer vorher festgelegten Menge eines flüssigen Polymermaterials gefüllt zu werden, um das betreffende piezoresistive sensitive Element (120), das innerhalb der besagten Öffnung untergebracht ist, vollständig zu bedecken, wobei das besagte flüssige Polymermaterial zum Aushärten geeignet ist, so dass ein Übertragungselement (160) bereitgestellt wird, um die externen Kräfte auf das piezoresistive sensitive Element (120) zu übertragen, welches aus dem besagten Hauptkörper (125) herausragt; insbesondere ist das elastische Material eine flexible gedruckte Leiterplatte ist, speziell eine Flex-PCB; und wobei insbesondere jeder Hauptkörper mit einer dritten Öffnung versehen ist, wobei die besagte dritte Öffnung derart angeordnet ist, um beim Gebrauch einen Temperatursensor zu beherbergen.

14. Eine Tonometerstruktur (100) gemäß Anspruch 13, wobei auf dem besagten Übertragungselement eine weitere Schicht aus Silikonkautschuk oder Gummi angeordnet ist, oder eine Schicht aus einem beliebigen biokompatiblen Material, welches dazu geeignet ist, während des Gebrauchs mit der Haut des Anwenders in Kontakt gebracht zu werden, um den Komfort des Patienten zu steigern.

15. Eine Tonometerstruktur (100) gemäß Anspruch 13, wobei auch ein Versteifungselement vorgesehen ist, insbesondere ein Versteifungselement aus einem steifen Kunststoffmaterial wie einem steifen PCB, welches an der besagten zweiten Seite des besagten tragbaren Stützteils angeordnet ist, an einem zugeordneten Hauptkörper, zum Beispiel an einer dem Hauptkörper in Bezug auf das tragbare Stützteil spiegelbildlich gegenüber liegenden Stelle, wobei die besagten Versteifungselemente und die besagten Hauptkörper während des Gebrauchs entlang einer axialen Richtung des besagten Arterienzweigs angeordnet sind; insbesondere ist auch ein Einstellmittel zum Einstellen des Abstandes der besagten Sensoreinheit von der besagten künstlichen Arterie vorgesehen, wobei das besagte Einstellmittel derart angeordnet ist, um die besagte Sensoreinheit von/zu dem besagten Arterienzweig zu entfernen/anzunähern, um zu veranlassen, dass der besagte Arterienzweig dadurch flach wird, insbesondere ist ein Steuermittel vorgesehen, das dazu ausgelegt ist, das detektierte Drucksignal zu lesen und es mit einem Referenzwert zu vergleichen, so dass in dem Fall, wenn der besagte Wert des besagten, detektierten Drucksignals höher ist als der besagte Referenzwert, eine Anzeige und/oder eine Schallquelle des besagten Tonometers dem Patient mitteilt, dass das detektierte Signal stark genug zur Detektion des arteriellen Drucks ist.

## Revendications

1. Structure de tonomètre (100) pour surveiller en continu la pression artérielle d'un patient pendant un temps déterminé, laquelle structure de tonomètre (100) comprend :
- un corps de support extracorporel (10) disposé de manière à être porté par un patient (50) ;
- une unité de détection (150) montée sur ledit corps de support extracorporel (10), ledit corps de support (10) et ladite unité de détection (150) étant configurés de façon que ladite unité de détection, lors de son utilisation, vienne au contact de la peau (50) dudit patient à proximité d'un trajet artériel (52), ladite unité de détection (150) ayant une pluralité de capteurs de pression piézorésistifs (20), chaque capteur de ladite pluralité étant configuré de façon à mesurer une variation de pression quand ladite unité de détection (150) vient au contact de la peau dudit utilisateur, et configuré pour délivrer un signal de mesure respectif (25), ledit signal de mesure (25) étant associé à une onde sphygmique respective d'une amplitude déterminée,
laquelle structure de tonomètre (100) comprend en outre :
- une unité de commande (30) associée à ladite pluralité de capteurs de pression (20) et disposée de manière à recevoir ladite pluralité de signaux de mesure (25), ladite unité de commande étant équipée d'un moyen de reconnaissance (32) qui est programmé avec un algorithme de reconnaissance configuré pour traiter ladite pluralité de signaux de mesure et comprenant les étapes suivantes :
- reconnaissance d'un signal de référence (26) parmi ladite pluralité de signaux de mesure (25), lequel signal de référence est associé à un capteur de référence respectif (21), et lequel signal de référence est le signal de mesure de ladite pluralité associé à l'onde sphygmique ayant l'amplitude la plus grande ;
- reconnaissance d'un signal limite (27) parmi la partie restante de ladite pluralité de signaux de mesure (25), ledit signal limite étant associé à un capteur de limite respectif (22), et lequel signal limite (27) est le signal de mesure (25) de ladite pluralité associé à l'onde sphygmique ayant l'amplitude la plus petite ;
- un moyen d'actionnement (34) disposé de manière à actionner ledit moyen de reconnaissance (32) à des instants prédéterminés (t1, t2, t3, tn), ledit moyen de reconnaissance (32) étant configuré pour mesurer un signal de mesure de référence (26) et pour identifier un capteur de référence relatif (21) à chacun desdits instants (t1, t2, t3, tn), de façon qu'aussi dans le cas d'un mouvement accidentel de ladite unité de détection (150) par rapport audit trajet artériel (52), un capteur de référence (21) soit toujours reconnu ;
ladite unité de commande (30) comprenant aussi un moyen formant programme (35) comprenant :
- un algorithme de filtration configuré pour réaliser une étape de traitement dudit signal limite (27) pour extraire une composante sphygmique et une composante de bruit à partir dudit signal limite (27) ;
- un algorithme de soustraction configuré pour réaliser une étape de soustraction de ladite composante de bruit dudit signal limite (27) à partir dudit signal de référence pour que soit obtenu un signal de référence corrigé qui est nettoyé des artéfacts et du bruit de fond ;
- un algorithme de transformation configuré pour réaliser une étape de traitement dudit signal de référence corrigé pour fournir une valeur en temps réel (40, 40', 40*) de la pression artérielle du patient.

2. Structure de tonomètre (100) selon la revendication 1, dans lequel ledit moyen formant programme est configuré pour exécuter aussi les étapes suivantes :
- combinaison, par un algorithme de fusion, dudit signal de référence et de ladite composante sphygmique dudit signal limite, ce qui donne un signal de référence amplifié, afin que soient augmentées les informations et la qualité dudit signal de référence lui-même ;
- traitement dudit signal de référence amplifié par ledit algorithme de transformation.

3. Structure de tonomètre (100) selon la revendication 1, dans lequel ledit moyen de reconnaissance est configuré pour traiter le spectre de fréquences desdits signaux de mesure (25) dans une bande de fréquences prédéterminée à laquelle la pression de signal appartient, ladite bande de fréquences prédéterminée appartenant à une bande de fréquence comprenant ladite fréquence cardiaque.

4. Structure de tonomètre (100) selon la revendication 1, dans lequel ledit moyen de reconnaissance (32) est configuré pour reconnaître une pluralité de signaux limites (27) par rapport audit signal de référence (26) dans la partie restante de ladite pluralité de capteurs (20), ledit moyen de reconnaissance étant fourni par ledit moyen formant programme (35) afin d'améliorer les informations dudit signal de référence (26), par un algorithme de fusion (36), et afin de déterminer ledit signal d'artéfact (28) devant être soustrait dudit signal de référence (26) par un algorithme de soustraction (37).

5. Structure de tonomètre (100) selon la revendication 1, dans lequel ladite unité de détection (150) comprend une pluralité de boîtiers (13), chaque boîtier de ladite pluralité de boîtiers étant disposé de manière à loger un capteur respectif (20), de façon qu'une unité de capteur soit formée (20a) ; en particulier chaque boîtier (13) étant fait en un matériau flexible qui est adapté pour transmettre les forces externes au capteur qui est logé à l'intérieur dudit boîtier (13).

6. Structure de tonomètre (100) selon la revendication 5, dans laquelle chaque boîtier (13) a une forme allongée en forme de boîte et est adapté pour loger un capteur allongé, ledit capteur allongé comprenant une base de support (20'a) et un élément sensitif (20c) connecté à ladite base de support (20'a).

7. Structure de tonomètre (100) selon la revendication 5, dans laquelle ledit matériau flexible est un caoutchouc de silicone, chaque boîtier (13) étant fait en un matériau rigide et disposé de manière à être rempli d'un matériau polymère fluide qui, une fois durci, est adapté pour transmettre les forces externes au capteur (20) contenu dans ledit boîtier fait en un matériau rigide, chaque boîtier (13) fait en un matériau rigide étant disposé de manière à loger un capteur qui est formé par ledit élément sensitif (20c) uniquement.

8. Structure de tonomètre (100) selon la revendication 7, dans laquelle ledit matériau rigide est un matériau céramique.

9. Structure de tonomètre (100) selon la revendication 7, dans laquelle ledit matériau polymère fluide est un caoutchouc de silicone.

10. Structure de tonomètre (100) selon la revendication 5, dans laquelle ladite pluralité d'unités de capteur (20a) est disposée en fonction d'un plan prédéterminé (2xN), dans laquelle 2xN est le nombre desdits éléments de capteur (20a), sur ledit corps de support, ladite unité de capteur (20a) étant disposée à une première extrémité (11a) dudit corps de support flexible (10) et dotée d'un connecteur d'alimentation (14) disposé à une deuxième extrémité (11b) opposée à ladite première extrémité (11a).

11. Structure de tonomètre (100) selon la revendication 6, dans laquelle ladite unité de détection en forme de boîte (150) comprend un seul boîtier (13) disposé de manière à loger une feuille munie de capteurs (13a) sur laquelle ladite pluralité de capteurs (20) sont disposés.

12. Structure de tonomètre (100) selon la revendication 1, dans laquelle ledit corps de support extracorporel (10) comprend, mis à part lesdits capteurs de pression (20), au moins un capteur auxiliaire configuré pour fournir d'autres données utiles, ledit ou chaque capteur auxiliaire étant choisi dans le groupe constitué par :
- un accéléromètre pour éliminer les artéfacts dus à un mouvement du patient (50) ;
- un capteur biochimique pour mesurer les électrolytes dans la peau, pour augmenter les données physiologiques ou diagnostiques du patient (50) ;
- un capteur de température pour compenser les changements de lecture intrinsèques des capteurs piézorésistifs ;
- un actionneur et un dispositif de commande dudit actionneur pour positionner plus aisément et obtenir un positionnement optimal au niveau dudit trajet artériel (52), ledit actionneur étant disposé de manière à appliquer sur ledit trajet artériel (52) une pression d'aplatissement adaptée au mieux pour détecter l'onde sphygmique ;
- ou une combinaison de ceux-ci ; en particulier comprenant un corps de base doté dudit ou de chaque autre capteur, ledit corps de base étant disposé de manière à être connecté de façon amovible à ladite structure de tonomètre afin de permettre la disposition desdits autres capteurs.

13. Structure de tonomètre (100) selon la revendication 1, comprenant :
- un corps de support extracorporel (10) fait en un matériau flexible, ledit corps de support extracorporel (10) ayant un premier côté (11) qui, lors de l'utilisation, peut être orienté en direction de la peau de l'utilisateur (50) et un deuxième côté (12) opposé au premier côté ;
- une pluralité de capteurs de pression piézorésistifs (120) disposés de manière à former des rangées (121), chaque capteur piézorésistif (120) de ladite pluralité étant disposé sur ledit premier côté (11) dudit corps de support extracorporel (10) ;
- un corps principal fait en un matériau rigide (125) disposé sur ledit premier côté (11) dudit corps de support extracorporel (10), ledit corps principal (10) ayant une pluralité d'ouvertures (113) au niveau de chacune desquelles, lors de l'utilisation, est disposé un élément sensitif piézorésistif respectif (120) de l'une desdites rangées, de façon qu'une unité de capteur (112) soit formée ;
- chaque ouverture (113) de ladite pluralité étant disposée de manière à être remplie par une quantité prédéterminée d'un matériau polymère fluide afin de couvrir complètement l'élément sensitif piézorésistif (120) respectif qui est logé à l'intérieur de ladite ouverture, ledit matériau polymère fluide étant adapté pour durcir de façon qu'un élément de transmission (160) soit disposé en étant agencé de manière à transmettre les forces externes à l'élément sensitif piézorésistif (120), qui fait saillie depuis ledit corps principal (125), en particulier ledit matériau polymère fluide étant un caoutchouc de silicone ; en particulier ledit matériau flexible étant une carte de circuit imprimé flexible, en particulier un circuit flex ; en particulier chaque corps principal étant aussi doté d'une troisième ouverture, ladite troisième ouverture étant disposée, lors de l'utilisation, de manière à loger un capteur de température.

14. Structure de tonomètre (100) selon la revendication 13, dans laquelle une autre couche de caoutchouc ou de caoutchouc de silicone est disposée sur ledit élément de transmission, ou une couche faite en un quelconque matériau biocompatible qui est adapté, lors de l'utilisation, pour être mis en contact avec la peau de l'utilisateur, est disposée, afin d'augmenter le confort du patient.

15. Structure de tonomètre (100) selon la revendication 13, dans lequel un élément raidisseur est également disposé, en particulier un élément raidisseur fait en une matière plastique rigide telle qu'un PCB rigide, qui est disposé sur ledit deuxième côté dudit corps de support extracorporel au niveau d'un corps principal respectif, c'est-à-dire à une position miroir opposée audit corps principal, par rapport audit corps de support extracorporel, lesdits corps raidisseur et lesdits corps principaux étant disposés, lors de l'utilisation, le long d'une direction axiale dudit trajet artériel ; en particulier un moyen d'ajustement étant aussi disposé pour ajuster la distance entre ladite unité de capteur et ladite artère artificielle, ledit moyen d'ajustement étant disposé pour éloigner/rapprocher ladite unité de capteur dudit trajet artériel, pour rendre plat ledit trajet artériel, en particulier un moyen de commande étant disposé, configuré pour lire ledit signal de pression détectée et pour le comparer à une valeur de référence de façon que, lorsque ladite valeur dudit signal de pression détectée est supérieure à ladite valeur de référence, un affichage et/ou un émetteur sonore dudit tonomètre communique au patient que le signal détecté est suffisamment fort pour détecter la pression artérielle.
